(19) Europäisches Patentamt
European Patent Office
Office européen des brevets

(11) Veröffentlichungsnummer: **0 162 224**
**B1**

(12) # EUROPÄISCHE PATENTSCHRIFT

(45) Veröffentlichungstag der Patentschrift: **31.10.90**

(21) Anmeldenummer: **85103305.0**

(22) Anmeldetag: **21.03.85**

(51) Int. Cl.$^5$: **C 07 D 207/335,**
C 07 D 231/12,
C 07 D 277/28,
C 07 D 307/58,
C 07 D 263/48,
C 07 D 271/06,
C 07 D 261/08,
C 07 D 307/56,
C 07 D 249/06, A 01 N 43/36,
A 01 N 43/56

(54) **Cyclohexenonderivate und ihre Verwendung zur Bekämpfung unerwünschten Pflanzenwuchses.**

(30) Priorität: **29.03.84 DE 3411530**
**17.08.84 DE 3430229**

(43) Veröffentlichungstag der Anmeldung:
**27.11.85 Patentblatt 85/48**

(45) Bekanntmachung des Hinweises auf die Patenterteilung:
**31.10.90 Patentblatt 90/44**

(84) Benannte Vertragsstaaten:
**BE CH DE FR GB IT LI NL**

(56) Entgegenhaltungen:
**EP-A-0 066 195**
**EP-A-0 071 707**
**EP-A-0 095 330**
**EP-A-0 125 094**
**EP-A-0 131 875**
**US-A-4 011 256**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Becker, Rainer, Dr.**
**Im Haseneck 22**
**D-6702 Bad Duerkheim (DE)**
Erfinder: **Jahn, Dieter, Dr.**
**Burgunderweg 8**
**D-6803 Edingen-Neckarhausen (DE)**
Erfinder: **Keil, Michael, Dr.**
**Fontanestrasse 4**
**D-6713 Freinsheim (DE)**
Erfinder: **Theobald, Hans, Dr.**
**Queichstrasse 6**
**D-6703 Limburgerhof (DE)**
Erfinder: **Spiegler, Wolfgang, Dr.**
**Westpreussenstrasse 5**
**D-6520 Worms 27 (DE)**
Erfinder: **Wuerzer, Bruno, Dipl.-Landwirt**
**Ruedigerstrasse 13**
**D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Cyclohexenonderivate sowie Herbizide, die diese Verbindungen als Wirkstoffe enthalten.

Es ist bekannt, Cyclohexenonderivate, die in 5-Stellung durch heterocyclische Reste substituiert sind, zur Bekämpfung von unerwünschten Gräsern in breitblättrigen Kulturen zu verwenden (DE—OS 24 39 104, DE—OS 31 23 312, EP—A—0 066 195, EP—A—0 071 707, US—A—4 011 256). Ihre herbizide Wirkung ist unbefriedigend. In den älteren nicht veröffentlichten Patentanmeldungen EP—A—0 125 094, EP—A—0 131 875 werden in 5-Stellung durch Heterocyclen substituierts Cyclohexenonderivate als Herbizide beschrieben, von denen eines in 5-Stellung durch Isoxazolyl substituiert ist.

Es wurde gefunden, daß Cyclohexenonderivate der Formel

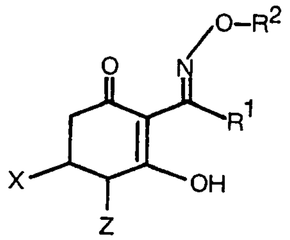

(I),

in der

$R^1$ Alkyl mit 1 bis 4 C-Atomen,

$R^2$ Alkyl mit 1 bis 4 C-Atomen, gegebenenfalls halogensubstituiertes Alkenyl mit 3 bis 5 C-Atomen oder Alkinyl mit 3 bis 5 C-Atomen,

X einen in 3-Stellung durch Halogen, $C_1$—$C_4$-Alkyl, $C_3$—$C_7$-Cycloalkyl, Di-$C_1$—$C_4$-alkylamino, $C_1$—$C_4$-Alkoxy, Phenyl, Phenoxy, Phenyl substituiert durch Halogen, $C_1$—$C_4$-Alkyl, $C_1$—$C_4$-Halogenalkyl, Phenoxy substituiert durch Halogen, $C_1$—$C_4$-Alkyl, $C_1$—$C_4$-Halogenalkyl substituierten Isoxazol-5-yl-Rest, und

Z Wasserstoff, Methoxycarbonyl, Ethoxycarbonyl, Methyl oder Cyano

bedeuten sowie die Salze dieser Verbindungen herbizid wirksam sind.

Die Verbindungen der Formel I können im tautormeren Formen auftreten, die alle vom Patentanspruch umfaßt werden.

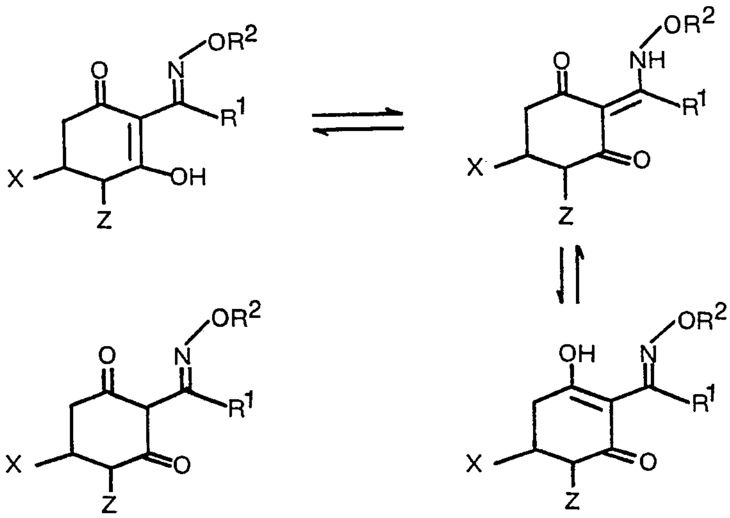

In Formel I bedeutet beispielsweise

$R^1$ verzweigtes oder unverzweigtes Alkyl mit 1 bis 4 C-Atomen, d.h. Methyl, Ethyl, n-Propyl, i-Propyl, n-Butyl, s-Butyl, i-Butyl, t-Butyl;

$R^2$ verzweigtes oder unverzweigtes Alkyl mit 1 bis 4 C-Atomen, gegebenenfalls halogensubstituiertes Alkenyl mit 3 bis 5 C-Atomen, wobei Halogen insbesondere Brom oder Chlor ist, oder Alkinyl mit 3 bis 5 C-Atomen, wie Methyl, Ethyl, n-Propyl, i-Propyl, n-Butyl, s-Butyl, i-Butyl, allyl, 1-Chlor-prop-1-en-3-yl, 2-Chlor-prop-1-en-3-yl, 1,2-Dichlor-prop-1-en-3-yl, 1,2-Dibrom-prop-1-en-3-yl, 1,1,2-Trichlor-prop-1-en-3-yl, Propargyl;

Z Wasserstoff, Methoxycarbonyl, Ethoxycarbonyl, Methyl oder Cyano;

X einen in 3-Stellung substituierten Isoxazol-5-yl-Rest, wobei dieser Rest durch Phenyl, Phenoxy, durch Halogen, $C_1$—$C_4$-Alkyl oder $C_1$—$C_4$-Halogenalkyl, insbesondere Chlor, Brom, Fluor, Methyl, Ethyl, Trifluormethyl, Difluormethyl, substituiertes Phenyl, wie 4-Chlorphenyl, 3,4-Dichlorphenyl, 3-Trifluormethylphenyl, 4-Methyl-phenyl, durch Halogen, $C_1$—$C_4$-Alkyl oder $C_1$—$C_4$-Halogenalkyl, insbesondere Chlor, Brom, Fluor, Methyl, Ethyl, Trifluormethyl, Difluormethyl substituiertes Phenoxy, Halogen, wie Fluor, Chlor, Brom oder Iod, Alkyl mit 1 bis 4 C-Atomen, wie Methyl, Ethyl, i-Propyl, t-Butyl,

Cycloalkyl mit 3 bis 7 C-Atomen, wie Cyclohexyl, Dialkylamino mit 1 bis 4 C-Atomen in einer Alkylgruppe, wie Dimethylamino, Diethylamino, Di-n-butylamino, Alkoxy mit 1 bis 4 C-Atomen, wie Methoxy, Ethoxy, i-Propoxy, n-Butoxy, s-Butoxy, t-Butoxy, substituiert sein kann.

Als Salze der Verbindungen der Formel I kommen landwirtschaftlich brauchbare Salze, beispielsweise die Alkalimetallsalze, wie Kalium- oder Natriumsalze, Erdalkalimetallsalze, wie Calcium, Barium- oder Magnesiumsalze, weiterhin Mangan-, Kupfer-, Zink- oder Eisensalze sowie Ammonium- oder Phosphoniumsalze in Betracht.

Bevorzugte Verbindungen der Formel I tragen als Substituenten X Isoxazol-5-yl, wobei dieser Rest durch Phenyl, Phenoxy, durch Halogen, $C_1$—$C_4$-Alkyl oder $C_1$—$C_4$-Halogenalkyl, insbesondere Chlor, Brom, Fluor, Methyl, Ethyl, Trifluormethyl, Difluormethyl, substituiertes Phenyl, wie 4-Chlorphenyl, 3,4-Dichlorphenyl, 3-Trifluormethylphenyl, 4-Methyl-phenyl, durch Halogen, $C_1$—$C_4$-Alkyl oder $C_1$—$C_4$-Halogenalkyl, insbesondere Chlor, Brom, Fluor, Methyl, Ethyl, Trifluormethyl, Difluormethyl, substituiertes Phenoxy, Halogen, wie Fluor, Chlor, Brom oder Iod, Alkyl, mit 1 bis 4 C-Atomen, wie Methyl, Ethyl, i-Propyl, t-Butyl, Cycloalkyl mit 3 bis 7 C-Atomen, wie Cyclohexyl, Dialkylamino mit 1 bis 4 C-Atomen in einer Alkylgruppe, wie Dimethylamino, Diethylamino, Di-n-butylamino, Di-n-butylamino, Alkoxy mit 1 bis 4 C-Atomen, wie Methoxy, Ethoxy, i-Propoxy, n-Butoxy, s-Butoxy, t-Butoxy, substituiert sein kann.

Weiterhin bevorzugt sind Cyclohexenonderivate der Formel I, bei denen Z Wasserstoff bedeutet.

Die Verbindungen der Formel I können z.B. durch Umsetzung von Verbindungen der Formel

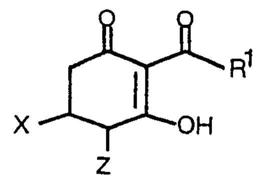

(II),

in der $R^1$, X und Z die obengenannten Bedeutungen haben, mit Hydroxylaminderivaten $R^2O—NH_3Y$, in der $R^2$ die obengenannten Bedeutungen hat und Y ein beliebiges Anion bedeutet, erhalten werden.

Man führt die Reaktion zweckmäßigerweise in heterogener Phase in einem inerten Verdünnungsmittel bei einer Temperatur zwischen 0 und 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium, Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung ds pH-Bereiches erfolgt zweckmäßigerweise durch Zusatz von Acetaten, beispielsweise Alkalimetallaceten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^2O—NH_3Y$, zugesezt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Sunden beendet, das Reaktionsprodukt kann dann durch Einengen der Mischung, Zugabe von Wasser und Extraktion mit einen unpolaren Lösungsmittel, wie Methylenchlorid und Abdestillieren des Lösungsmittels unter vermindertem Druck isoliert werden.

Die Verbindung der Formel I können außerdem durch Umsetzen der Verbindungen der Formel II mit Hydroxylaminen der Formel $R^2O—NH_2$, in der $R^2$ die obengenannten Bedeutungen hat, in inerten Verdünnungsmitteln bei einer Temperatur zwischen 0°C und dem Siedepunkt des Reaktionsgemisches, insbesondere zwischen 15 und 70°C, erhalten werden. Gegebenenfalls kann das Hydroxylamin als wäßrige Lösung eingesetzt werden.

Geeignete Lösungsmittel für diese Umsetzung sindbeispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, dichlorethan, Ester, wie Essigsäureethylester, Nitrile, wie Acetonitril, cyclische Ether, wie Tetrahydrofuran.

Die Verbindungen der Formel I können weiterhin durch Umsetzen von Verbindungen der Formel II mit unsubstituiertem Hydroxylammoniumsalz $NH_2OH.HY$, wobei Y die obige Bedeutung hat, zu einem entsprechenden Oxim umgesetzt und dann O-alkyliert werden. Dabei muß man die Neigung der als Zwischenprodukte gebildeten Oxime zu unerwünschten Cyclisierungsreaktionen beachten; durch geeignete Hilfsstoffe und Reaktionsbedingungen läßt sich dies beeinflussen.

Geeignete Lösungsmittel sind die für die Umsetzung der Verbindungen der Formel II mit Hydroxylaminen aufgeführten Lösungsmittel, geeignete Hilfsbasen sind die für die Umsetzung der Verbindungen der Formel II mit Hydroxylaminderivaten der Formel $R^2—O—NH_3Y$ genannten basischen Substanzen, wobei allerdings die doppelte Menge an Base erforderlich ist.

Die Alkalimetallsalze der Verbindungen der Formel I können durch Behandeln dieser Verbindungen mit Natrium- oder Kaliumhydroxid in wäßriger Lösung oder in einem organischen Lösunkgsmittel, wie

Methanol, Ethanol, Aceton, erhalten werden. Auch Natrium- und Kaliumalkoholate können als Basen dienen.

Die anderen Metallsalze, z.B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium- und Phosphoniumsalze können durch Umsetzung von Verbindungen der Formel I mit Ammonium- oder Phosphoniumhydroxiden gegebenenfalls in wäßriger Lösung hergestellt werden.

Die Verbindungen der Formel II können aus Cyclohexan-1,3-dionen der Formel IV, die auch in den tautomeren Formeln IVa und IVb vorliegen können,

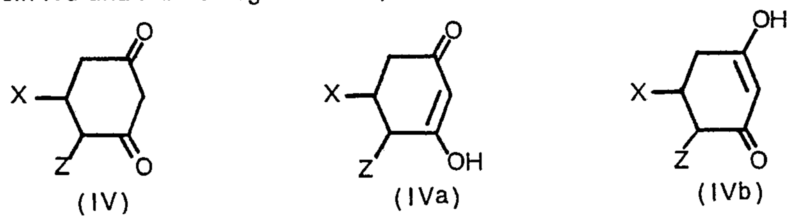

(IV) (IVa) (IVb)

nach literaturbekannten Methoden hergestellt werden (Tetrahedron Letters, *29*, 2491 (1975)).

Es ist auch möglich, Verbindungen der Formel II über die Zwischenstufe der Enolester, die bei der Umsetzung von Verbindungen der Formel IV eventuell als Isomerengemische anfallen und in Gegenwart von Imidazol- oder Pyridinderivaten umgelagert werden (JP—OS 79/063052), herzustellen.

Zu den Verbindungen der Formel IV gelangt man nach literaturbekannten Verfahren, wie dies aus folgendem Schema hervorgeht:

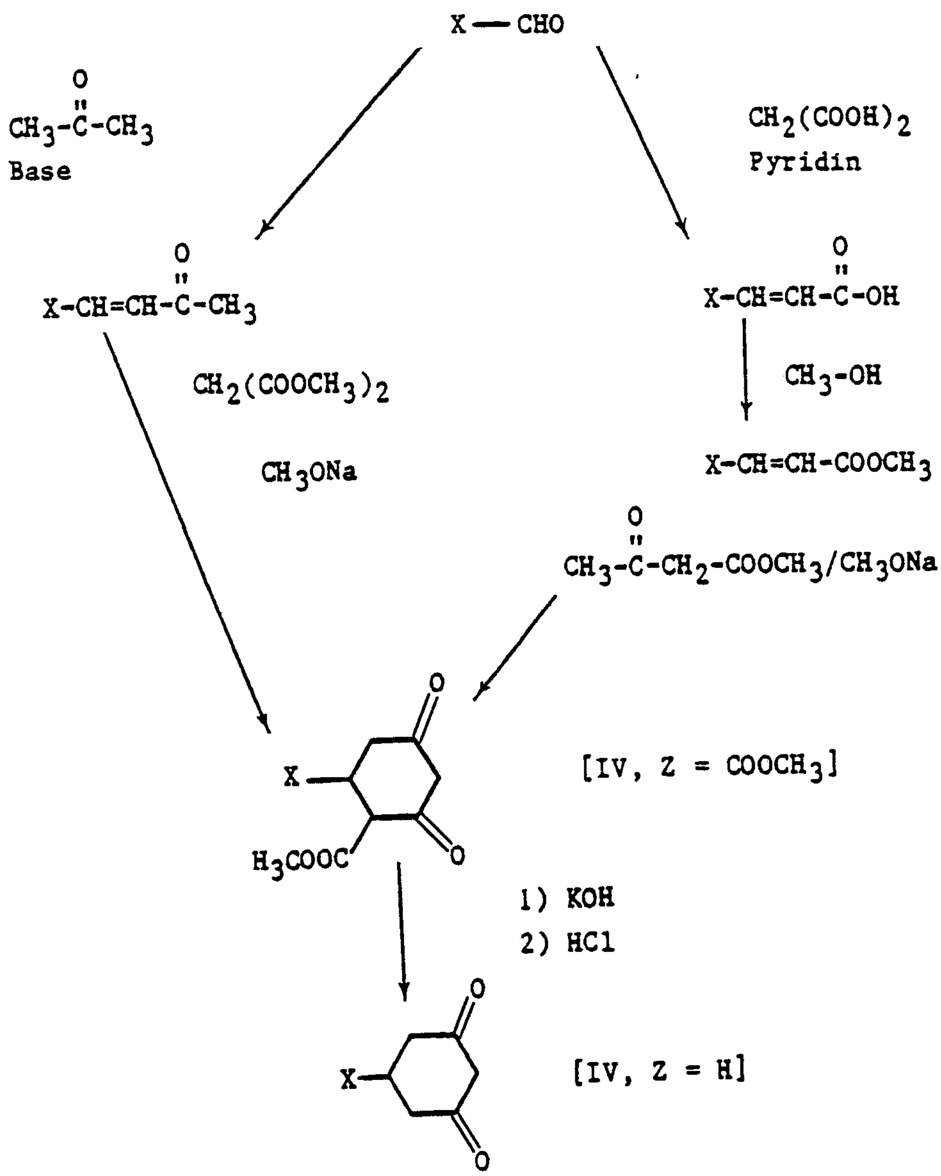

Es versteht sich, daß die Art der Einführung des Substituenten X abweichend vom Vorstehend von Fall zu Fall geändert werden kann bzw. ein eingeführter Substituent X noch verändert werden kann.

Nach diesen Verfahren werden die folgenden Verbindungen der Formel I hergestellt. Nicht näher charakterisierte Verbindungen können unter sinngemäßer Abwandlung der vorstehenden Vorschrift erhalten werden. Aufgrund struktureller Ähnlichkeit kann erwartet werden, daß sie eine gleichartige biologische Wirkung aufweisen.

| Verbindung-Nr. | $R^1$ | $R^2$ | X | Z | Fp [°C]/$n_D$ |
|---|---|---|---|---|---|
| 78 | n-$C_3H_7$ | $C_2H_5$ | 3-Phenyl-isoxazol-5-yl | $COOCH_3$ | zähes Öl |
| 79 | n-$C_3H_7$ | $CH_2CH{=}CH_2$ | 3-Phenyl-isoxazol-5-yl | $COOCH_3$ | zähes Öl |
| 80 | n-$C_3H_7$ | $C_2H_5$ | 3-Phenyl-isoxazol-5-yl | H | 93—97 |
| 81 | n-$C_3H_7$ | $CH_2CH{=}CH_2$ | 3-Phenyl-isoxazol-5-yl | H | 71—73 |
| 82 | n-$C_3H_7$ | $CH_2CH{=}CHCl$ | 3-Phenyl-isoxazol-5-yl | H | |
| 104 | n-$C_3H_7$ | $C_2H_5$ | 3-Methyl-isoxazol-5-yl | H | 78—80 |
| 105 | n-$C_3H_7$ | $CH_2CH{=}CH_2$ | 3-Methyl-isoxazol-5-yl | H | 72—74 |
| 106 | n-$C_3H_7$ | cis-$CH_2CH{=}CHCl$ | 3-Methyl-isoxazol-5-yl | H | 63—65 |
| 107 | n-$C_3H_7$ | trans-$CH_2CH{=}CHCl$ | 3-Methyl-isoxazol-5-yl | H | 96—98 |
| 108 | n-$C_3H_7$ | $CH_2CH{=}CHCl$ | 3-Methyl-isoxazol-5-yl | H | 83—85 |
| 133 | n-$C_3H_7$ | $CH_2{—}CH{=}CH_2$ | 3-Methylisoxazol-5-yl | $COOCH_3$ | 93—95 |
| 134 | n-$C_3H_7$ | $C_2H_5$ | 3-Methylisoxazol-5-yl | $COOCH_3$ | 130—131 |

Die Cyclohexenonderivate der Formel I und ihre Salze können beispielsweise in Form von direkt versprühbaen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittleren Naphthalinen oder Methanol, Ethanol, Propanol, Butanol, Chloroform, Tetrachlorkohlenstoff, Cyclohexanol, Cyclohexanon, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, dispergier- oder Emulgiermittel in Wasser homogenisiert werden. Es können aber auch aus wirksamer Substanz Netz-, Haft-, dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalin-derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Säubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

IV. 20 Gewichtsteile des Wirkstoffs Nr. 79 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 81 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichsteile des Wirkstoffs Nr. 78 werrden mit 97 Gewichtsteilen feinteiligem Kaolin vermischt. Man erhält auf diese Weise ein Stäubemittel, das 3 Gewichtsprozent des Wirkstoffs enthält.

Die Applikation kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Moglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstrumsstadium 0,025 bis 3 kg/ha, vorzugsweise 0,1 bis 1 kg/ha.

Die Wirkung der Cyclohexenon-Derivate der Formel I auf das Pflanzenwachstum läßt sich durch Gewächschausversuche zeigen:

Als Kulturgefäße dienen Plastikblumentöpfe mit 300 $cm^3$ Inhalt und lehmiem Sand mit etwa 1,5% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der nachauflaufbehandlung zieht man die Testpflanzen je nach Wuchsform erst bis zu einer Wuchshöhe von 3 bis 15 cm an und behandelte sie danach. Die Sojapflanzen werden in einem mit Torfsmull (peat) angereichterten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,25, 0,5 und 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dier Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Avena fatua (Flughafer), Avena sativa (Hafer), Bromus inermis (Unbegrannte Trespe), Digitaria sanguinalis (Blutfingerhirse), Echinochloa crus-galli (Hühnerhirse), Glycine max (Sojabohnen), Lolium multiflorum (Ital. Raygras), Setaria italica (Kolbenhirse), Sinapis alba (Weißer Senf), Sorghum halepense (Sudangras; Wilde Mohrenhirse), Triticum aestivum (Weizen), Zea mays (Mais).

Nachauflaufanwendung

Die beispielhaft ausgewählten Verbindungen Nr. 78, 79 und 81 bekämpfen grasartige unerwünschte Pflanzen selektiv in Weizen mit 1,0 kg Wirkstoff/ha. Bei der Bekämpfung von Schadgräsern in breiblättrigen Kulturen, z.B. in Sojabohnen, ist beispielsweise die Verbindung Nr. 108 ser gut wirksam.

In Anbetracht des erfaßbaren Wirkungsspektrums zur Umkrautbekämpfung, der Verträglichkeit für Kulturpflanzen oder der erwünschten Beeinflussung des Wachstrums derselben sowie angesichts der Vielfalt der Applikationsmethoden können die Cyclohexenonderivate der Formel I in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Annanas comosus | Ananas |
| Arachis hypogaea | Erdnuß |
| Asparagus officinalis | Spargel |
| Beta vulgaris spp. altissima | Zuckerrübe |
| Beta vultaris spp. rapa | Futterrübe |
| Beta vultaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Ruben |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojarbohne |
| Gossypium hirsutum (Gossypium arboreum Gossypium herbaceum Gossypium vitifolium) | Baumwolle |
| Helianthus annuus | Sonnenblume |

| Botanischer Name | Deutscher Name |
|---|---|
| Helianthus tubeross | Topinambur |
| Hevea brasiliensis | Parakauschukbaum |
| Hordeum vulgare | Gerste |
| Humulus lupulkus | Hopfen |
| Ipomoea batatas | Süßkartoffeln |
| Juglans regia | Walnußbaum |
| Lactua sativa | Kopfsalat |
| Lens culinaris | Linse |
| Linum usitatissium | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |

| Botanischer Name | Deutscher Name |
|---|---|
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kihbohne |
| Vitis vinifera | Weinrebe |
| Zea mays | Mais (Unterblattspritzung) |

Zur Verbreiterung des Wirkungspektrums und zur Erzielung synergistischer Effekte können die Cyclohexenonderivate der Formel I und ihre Salze mit zahlreichen Vertratern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexanonderivate anderer Struktur und andere herbizide Wirkstoffe in Betracht.

Außerdem kann es von Nutzen sein, die Verbindungen der Formel I allein oder in Kombination mit anderen Herbiziden, auch noch mit weiteren Pflanzenschutzmitteln gemischt gemeinsam auszubringen, beispielsweise mit Mitteln zum Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden.

**Patentansprüche**

1. Cyclohexenonderivate der Formel

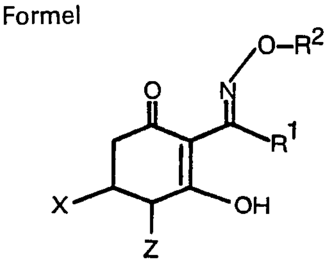

(I),

in der

$R^1$ Alkyl mit 1 bis 4 C-Atomen,

$R^2$ Alkyl mit 1 bis 4 C-Atomen, gegebenenfalls halogensubstituiertes Alkenyl mit 3 bis 5 C-Atomen oder Alkinyl mit 3 bis 5 C-Atomen,

X einen in 3-Stellung durch Halogen, $C_1$—$C_4$-Alkyl, $C_3$—$C_7$-Cycloalkyl, Di-$C_1$—$C_4$-alkylamino, $C_1$—$C_4$-Alkoxy, Phenyl, Phenoxy, Phenyl substituiert durch Halogen, $C_1$—$C_4$-Alkyl, $C_1$—$C_4$-Halogenalkyl, Phenoxy substituiert durch Halogen, $C_1$—$C_4$-Alkyl, $C_1$—$C_4$-Halogenalkyl substituierten Isoxazol-3-yl-Rest, und

Z Wasserstoff, Methoxycarbonyl, Ethoxycarbonyl, Methyl oder Cyano bedeuten, sowie die Salze dieser Verbindungen.

2. Cyclohexenonderivate der Formel I gemäß Anspruch 1, dadurch gekennzeichnet, daß Z Wasserstoff bedeutet.

3. Verfahren zur Herstellung eines Cyclohexenonderivates der Formel I gemäß Anspruch 1, dadurch gekennzeichnet, daß man eine Verbindung der Formel

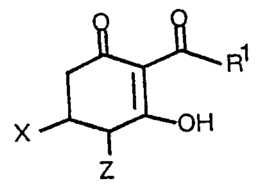

(II),

in der $R^1$, Z und X die in Anspruch 1 genannten Bedeutungen haben,

a) mit einem Hydroxylaminderivat der Formel $R^2ONH_3Y$, in der $R^2$ die im Anspruch 1 genannte Bedeutung hat und Y ein beliebiges Anion bedeutet, in einem inerten Lösungsmittel gegebenenfalls unter Zugabe einer Hilfsbase umsetzt, oder

b) mit einem gegebenenfalls in wäßriger Lösung vorliegenden Hydroxylamin der Formel $R^2ONH_2$, wobei $R^2$ die obige Bedeutung hat, gegebenenfalls unter Zugabe eines Lösungsmittels umgsetzt, oder

c) mit unsubstituiertem Hydroxylammoniumsalz $NH_2OH \cdot HY$, wobei Y ein beliebiges Anion bedeutet, unter Zugabe von Lösungsmittel und Hilfsbase umsetzt und das so erhaltene Oxim mit einem Alkylierungsmittel $R^2$—Y', wobei Y' eine Abgangsgruppe bedeutet und $R^2$ die im Anspruch 1 genannte Bedeutung hat, alkyliert.

4. Herbizid, enthaltend ein Cyclohexenonderivat der Formel I gemäß Anspruch 1 oder ein Salz einer solchen Verbindung.

5. Herbizid, enthaltend inerte Zusatzstoffe und ein Cyclohexenonderivat der Formel I gemäß Anspruch 1 oder ein Salz einer solchen Verbindung.

6. Verfahren zur Bekämpfung unerwünschten Pflanzenwuchses, dadurch gekennzeichnet, daß man die unerwünschten Pflanzen oder von unerwünschtem Pflanzenwachstum freizuhaltende Fläche mit einer herbizid wirksamen Menge eines Cyclohexenonderivates der Formel I gemäß Anspruch 1 oder eines Salzes einer solchen Verbindung behandelt.

7. Verfahren gemäß Anspruch 6, dadurch gekennzeichnet, daß die Menge an Cyclohexenonderivat der Formel I 0,025 bis 3 kg/ha beträgt.

8. Cyclohexenonderivat gemäß Formel I im Anspruch 1, in der $R^1$ Propyl, $R^2$ Ethyl, Z Wasserstoff und X 3-Methylisoxazol-5-yl bedeutet.

9. Cyclohexenonderivat gemäß Formel I in Anspruch 1, in der $R^1$ Propyl, $R^2$ Ethyl, Z Wasserstoff und X 3-Phenylisoxazol-5-yl bedeutet.

**Revendications**

1. Dérivés de cyclohéxénone de formule I

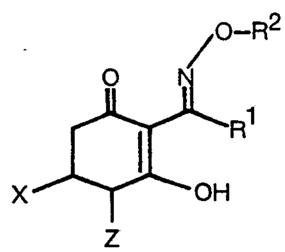

(I),

dans laquelle

$R^1$ représente alkyle de 1 à 4 atomes C

$R^2$, alkyle de 1 à 4 atomes C, alcényle de 3 à 5 atomes C ou alcynyle de 3 à 5 atomes C, éventuellement halogénosubstitué,

X, un reste isoxazol-5-yle substitué en position 3 par halogène, alkyle en $C_1$—$C_4$, cycloalkyle en $C_3$—$C_7$, dialkyl (en $C_1$—$C_4$)-amino alcoxy en $C_1$—$C_4$, phényle, phénoxy, phényle substitué par halogène, alkyle en $C_1$—$C_4$, halogénoalkyle en $C_1$—$C_4$, phénoxy substitué par halogène, alkyle en $C_1$—$C_4$, halogénoalkyle en $C_1$—$C_4$, et

Z, représente hydrogène, méthoxycarbonyle, éthoxycarbonyle, méthyle ou cyano ainsi que les sels de ces composés.

2. Dérivés de cyclohéxénone de formule I selon la revendication 1, caractérisés par le fait que Z représente hydrogène.

3. Procédé de préparation d'un dérivé de cyclohéxénone de formule I selon la revendication 1, caractérisé par le fait que l'on fait réagir un composé de la formule

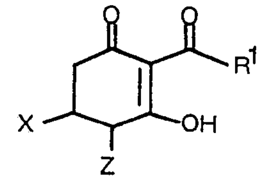

(II),

dans laquelle $R^1$, Z et X ont les significations indiquées dans la revendication 1:

a) avec un dérivé d'hydroxylamine de la formule $R^2ONH_3Y$, dans laquelle $R^2$ a la signification indiquée dans la revendication 1 et Y représente un anion quelconque, dans un solvant inerte, éventuellement avec addition d'une base auxiliaire, ou

b) avec une hydroxylamine de la formule $R^2ONH_2$, se trouvant eventuellement en solution aqueuse, $R^2$ ayant la signification ci-dessus, éventuellement avec addition d'un solvant ou

c) avec un sel d'hydroxylammonium non substitué NH2OH·HY, Y représentant un anion quelconque, avec addition de solvant et de base auxiliaire, et on alkyle l'oxime ainsi obtenu avec un agent d'alkylation $R^2$—Y', Y' représentant un groupe éliminable et $R^2$ ayant la signification donnée dans la revendication 1.

4. Herbicide contenant un dérivé de cyclohéxénone de formule I selon la revendication 1 ou un sel d'un tel composé.

5. Herbicide contenant des additivs inertes et un dérivé de cyclohéxénone de formule I selon la revendication 1 ou un sel d'un tel composé.

6. Procédé de lutte contre une croissance indésirable des plantes, caractérisé par le fait qu'on traite les plantes indésirables, ou les surfaces à maintenir exemptes d'une croisance indésirable des plantes avec une quantité efficace du point de vue herbicide d'un dérivé de cyclohéxénone de formule I selon la revendication 1 ou un sel d'un tel composé.

7. Procédé selon la revendication 6, caractérisé par le fait que la dose de dérivé de cyclohéxénone de formule I est de 0,025 à 3 kg/ha.

8. Dérivé de cyclohéxénone de formule I selon la revendication 1 dans laquelle $R^1$ représente propyle, $R^2$, éthyle, Z, hydrogène et X, 3-méthylisoxazol-5-yle.

9. Dérivé de cyclohéxénone de formule I selon la revendication 1 dans laquelle $R^1$ représente propyle, $R^2$, éthyle, Z, hydrogène et X, 3-phénylisoxazol-5-yle.

**Claims**

1. A cyclohexenone derivative of the formula

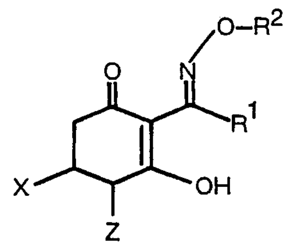

(I),

where $R^1$ is alkyl of 1 to 4 carbon atoms, $R^2$ is alkyl of 1 to 4 carbon atoms, unsubstituted or halogen-substituted alkenyl of 3 to 5 carbon atoms or alkynyl of 3 to 5 carbon atoms, X is an isoxazol-5-yl radical which is substituted in the 3-position by halogen, $C_1$—$C_4$-alkyl, $C_3$—$C_7$-cycloalkyl, di-$C_1$—$C_4$-alkylamino, $C_1$—$C_4$-alkoxy, phenyl, phenoxy, phenyl substituted by halogen, $C_1$—$C_4$-alkyl, $C_1$—$C_4$-haloalkyl, or phenoxy substituted by halogen, $C_1$—$C_4$-alkyl or $C_1$—$C_4$-haloalkyl, and Z is hydrogen, methoxycarbonyl, ethoxycarbonyl, methyl or cyano, and the salts thereof.

2. A cyclohexenone derivative of the formula I as claimed in claim 1, where Z is hydrogen.

3. A process for the preparation of a cyclohexenone derivative of the formula I as claimed in claim 1, wherein a compound of the formula

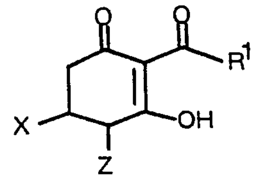

(II),

where $R^1$, Z and X have the meanings given in claim 1, is reacted with

a) a hydroxylamine derivative of the formula $R^2ONH_3Y$, where $R^2$ has the meanings given in claim 1 and Y is any anion, in an inert solvent with or without the addition of an auxiliary base, or

b) a hydroxylamine — if desired, in aqueous solution — of the formula $R^2ONH_2$, where $R^2$ has the above meanings, with or without the addition of a solvent, or

c) an unsubstituted hydroxylammonium salt $NH_2OH.HY$, where Y is any anion, with the addition of a solvent and an auxiliary base, and the oxime thus obtained is alkylated with an alkylating agent $R^2$—Y', where Y' is a leaving group and $R^2$ has the meanings given in claim 1.

4. A herbicide containing a cyclohexenone derivative of the formula (I) as claimed in claim 1, or a salt thereof.

5. A herbicide containing inert additives and a cyclohexenone derivative of the formula I as claimed in claim 1, or a salt thereof.

6. A method for controlling undesired plant growth, wherein the undesired plants or the area to be kept free from undesired plant growth are or is treated with a herbicidally effective amount of a cyclohexenone derivative of the formula I as claimed in claim 1, or a salt thereof.

7. A process as claimed in claim 6, wherein the amount of cyclohexenone deivative of the formula I is from 0.025 to 3 kg/ha.

8. A cyclohexenone derivative as in formula I in claim 1, where $R^1$ is propyl, $R^2$ is ethyl, Z is hydrogen and X is 3-methylisoxazol-5-yl.

9. A cyclohexenone derivative as in formula I in claim 1, where $R^1$ is propyl, $R^2$ is ethyl, X is hydrogen and X is 3-phenylisoxazol-5-yl.